# EUROPÄISCHE PATENTSCHRIFT

(11) **EP 2 338 437 B1**
(45) Veröffentlichungstag und Bekanntmachung des Hinweises auf die Patenterteilung: **05.11.2014**
(21) Anmeldenummer: 10015162.0
(22) Anmeldetag: 01.12.2010
(51) Int. Cl.: A61B 19/00, F16M 11/04, F16F 1/02, A61B 17/00

(54) **Haltevorrichtung für medizinische Instrumente**
Holder for medicinal instruments
Dispositif de retenue pour instruments médicaux

(30) Priorität: 23.12.2009 DE 102009060495
(43) Veröffentlichungstag der Anmeldung: 29.06.2011
(73) Patentinhaber: Karl Storz GmbH & Co. KG, 78532 Tuttlingen (DE)
(72) Erfinder: Stefan, Jochen, 78532 Tuttlingen (DE); Bacher, Uwe, 78532 Tuttlingen (DE)
(74) Vertreter: Fugmann, Winfried

(56) Entgegenhaltungen:
- EP-A1- 1 586 925
- WO-A1-03/099152
- WO-A1-2007/054327
- DE-B3- 10 314 156
- US-A- 5 436 542
- US-B1- 6 371 463

## Beschreibung

Die Erfindung betrifft eine Haltevorrichtung für medizinische Instrumente, mit einem Tragarm, an dem mindestens ein medizinisches Instrument festlegbar ist, mit mindestens einem Gelenk zur Positionierung des Tragarms sowie mit einem Gewichtsentlastungssystem zur Kompensierung der Tragarmlast.

Derartige Haltevorrichtungen sind bei der Durchführung chirurgischer Eingriffe häufig erforderlich, um medizinische Instrumente verschiedenster Art, wie beispielsweise Retraktoren, Kameras oder Endoskope, über einen längeren Zeitraum in einer bestimmten Position zu halten. Durch die gelenkige Ausgestaltung der Haltevorrichtungen ist es für den Chirurgen möglich, das von dem Tragarm gehaltene medizinische Instrument exakt zu positionieren und die eingestellte Position der Haltevorrichtung durch Arretieren des Gelenks bzw. der Gelenke des Tragarms zu fixieren.

Um dem Operateur die Bedienung einer solchen mit einem medizinischen Instrument bestückten Haltevorrichtung zu erleichtern, werden diese Haltevorrichtungen häufig mit einem Gewichtsentlastungssystem zur Kompensierung der Tragarmlast der Haltevorrichtung versehen. Das Gewichtsentlastungssystem, meist in der Form von Gegengewichten oder Federpaketen, soll das Eigengewicht des Tragarms nebst den daran festgelegten medizinischen Instrumenten ausbalancieren, so dass der Operateur die Haltevorrichtung nahezu kraftfrei in jede gewünschte Position verschwenken kann.

Eine gattungsgemäße Haltevorrichtung mit einem Gewichtsentlastungssystem ist beispielsweise aus der DE 10 2005 054 010 A1 oder der korrespondierenden WO 2007/054327 A1 bekannt. Bei dieser bekannten Haltevorrichtung besteht das Gewichtsentlastungssystem aus einer Linearfederanordnung mit mehreren parallel zueinander angeordneten Federblöcken, die über einen Kurventräger am Schwenkgelenk des Tragarms angreifen. Aus dem US Patent US 5,436,542 A1 ist eine Kamerahalterung mit Federn mit konstanter Rückstellkraft bekannt.

Mit dieser aus der DE 10 2005 054 010 A1 bekannten Federanordnung ist es zwar möglich, die Haltevorrichtung im Wesentlichen kräftefrei zu verstellen, jedoch weist diese Federanordnung wie auch die aus der Praxis bekannten Konstruktionen mit Gegengewichten als Gewichtsentlastungssysteme den Nachteil auf, dass diese Systeme sehr großvolumig bauen und darüber hinaus ein hohes Gewicht aufweisen, so dass diese bekannten Haltevorrichtungen kaum nach jedem Einsatz demontiert und zu Reinigungszwecken autoklaviert werden können.

Davon ausgehend liegt der Erfindung die **Aufgabe** zugrunde, eine Haltevorrichtung für medizinische Instrumente der eingangs genannten Art zu schaffen, deren Gewichtsentlastungssystem bei kompakter und leichter Bauweise zu Reinigungszwecken zusammen mit dem Tragarm vollständig autoklavierbar ist.

Die **Lösung** dieser Aufgabenstellung ist erfindungsgemäß dadurch gekennzeichnet, dass das Gewichtsentlastungssystem als mit dem Tragarm gekoppeltes Rückstellelement aus einem im Wesentlichen eine Plateau-Spannung aufweisenden Material ausgebildet ist.

Werkstoffe, die eine Plateau-Spannung aufweisen, zeichnen sich dadurch aus, dass diese Werkstoffe im Spannungs-Dehnungs-Diagramm, das die Längenänderung (Dehnung) eines Werkstoffs in der Abhängigkeit von einer am Werkstoff angreifenden Zugkraft (Zugspannung) darstellt, ein im Wesentlichen horizontal verlaufendes Spannungsplateau aufweisen. Dies bedeutet, dass in diesem bestimmten, das Plateau bildenden Bereich eine Längenänderung (Dehnung) des Werkstoffs erzielbar ist, ohne dass eine höhere oder zumindest nur geringfügig höhere Kraft (Zugspannung) aufgebracht werden muss als vor und hinter dem Spannungsplateau.

Durch die erfindungsgemäße Ausbildung des Gewichtsentlastungssystems als am Tragarm angreifendes Rückstellelement ist es möglich, das Gewichtsentlastungssystem kompakt und leicht zu bauen, so dass die gesamte Haltevorrichtung leicht demontierbar und zu Reinigungszwecken autoklavierbar ist.

Gemäß einer praktischen ausführungsform der Erfindung wird vorgeschlagen, dass das eine Plateau-Spannung aufweisende Material des Rückstellelements eine CuZnAl-, FeNi-CoTi- oder NiTi-Legierung ist.

Mit einer bevorzugten Ausführungsform der Erfindung wird vorgeschlagen, dass das eine Plateau-Spannung aufweisende Material eine Formgedächtnis-Legierung ist.

Formgedächtnis-Legierungen zeichnen sich dadurch aus, dass sie nach einer relativ starken Verformung ihre ursprüngliche Gestalt wieder einnehmen können. Im Zusammenhang mit einer Formgedächtnis-Legierung bedeutet dies, dass der im Bereich seines Spannungsplateaus ohne zusätzlichen Kraftaufwand gedehnte Werkstoff nach dem Abstellen der Zugspannung wieder seine ursprüngliche Form annimmt.

Gemäß einer praktischen Ausführungsform der Erfindung wird vorgeschlagen, dass die das Rückstellelement bildende Formgedächtnis-Legierung eine NiTi-Legierung, insbesondere NiTiNol, ist. Die intermetallische Legierung NiTiNol zeichnet sich durch ein Spannungsplateau im Bereich einer Dehnung von 1 % bis 8 % aus. Dies bedeutet, dass ein auf 1 % Dehnung vorgespanntes NiTiNol Element ohne zusätzlichen Kraftaufwand bis auf 8 % gedehnt werden kann, wobei das NiTiNol Element zusätzlich aufgrund seiner Formgedächtniseigenschaft nach dem Abstellen der Zugspannung wieder seine ursprüngliche Länge einnimmt.

Um eine weitestgehend kraftfreie Betätigung des Tragarms zu ermöglichen wird erfindungsgemäß vorgeschlagen, dass das Rückstellelement durch die Tragarmlast bis auf das Erreichen der Plateau-Spannung vorgespannt ist, so dass die weitere Dehnung des Rückstellelements direkt in den kraftlosen Bereich des Spannungsplateaus fällt.

Gemäß einer praktischen Ausführungsform der Erfindung wird vorgeschlagen, dass das Rückstellelement das mindestens eine Gelenk überspannend mit dem Tragarm gekoppelt ist, so dass das Rückstellelement durch die Betätigung des Gelenks gespannt oder wieder entspannt wird.

Mit einer ersten praktischen Ausführungsform zur Ausbildung des Rückstellelements wird vorgeschlagen, dass das Rückstellelement als Seilzug ausgebildet ist.

Gemäß einer alternativen zweiten Ausführungsform wird vorgeschlagen, dass das Rückstellelement als Federelement ausgebildet ist.

Vorteilhafterweise ist der das Gewichtsentlastungssystem bildende Seilzug so mit dem Tragarm gekoppelt, dass der Seilzug mit einem Ende an einem ersten Tragarmteil gelagert ist und mit dem anderen Ende an einem über das mindestens eine Gelenk gegenüber dem ersten Tragarmteil verstellbaren zweiten Tragarmteil gelagert ist.

Weiterhin wird mit der Erfindung vorgeschlagen, dass der Seilzug über ein biegsames Element, beispielsweise eine Gliederkette, an dem verstellbaren Tragarmteil gelagert ist. Die Kopplung des Seilzugs mit dem verstellbaren Tragarmteil über die Gliederkette ermöglicht eine gleichmäßige und ruckfreie Übertragung der von dem verschwenkbaren Tragarmteil ausgeübten Kräfte auf den das Gewichtsentlastungssystem bildenden Seilzug.

Schließlich wird mit der Erfindung vorgeschlagen, dass der Seilzug in einem Führungsrohr gelagert ist, um eine sichere und störungsfreie Lagerung und Führung des Seilzuges zu gewährleisten.

Gemäß einer bevorzugten Ausführungsform der Erfindung wird vorgeschlagen, dass das Führungsrohr axialverschiebbar am Tragarm gelagert ist, so dass die Vorspannung des Seilzugs auf die Plateau-Spannung vorteilhafterweise über das axialverschiebbare Führungsrohr einstellbar ist, in dem der Seilzug gelagert ist.

Weitere Merkmale und Vorteile der Erfindung ergeben sich anhand der zugehörigen Zeichnungen, in denen ein Ausführungsbeispiel einer erfindungsgemäßen Haltevorrichtung für medizinische Instrumente nur beispielhaft dargestellt ist, ohne die Erfindung auf dieses Ausführungsbeispiel zu beschränken. In den Zeichnungen zeigt:
- Fig. 1: eine Haltevorrichtung für medizinische Instrumente gemäß dem Stand der Technik;
- Fig. 2: eine Prinzipskizze des Aufbaus einer erfindungsgemäßen Haltevorrichtung für medizinische Instrumente;
- Fig. 3: eine geschnittene Seitenansicht eines Teilbereichs einer erfindungsgemäßen Haltevorrichtung und
- Fig. 4: eine vergrößerte Darstellung des Details IV gemäß Fig. 3.

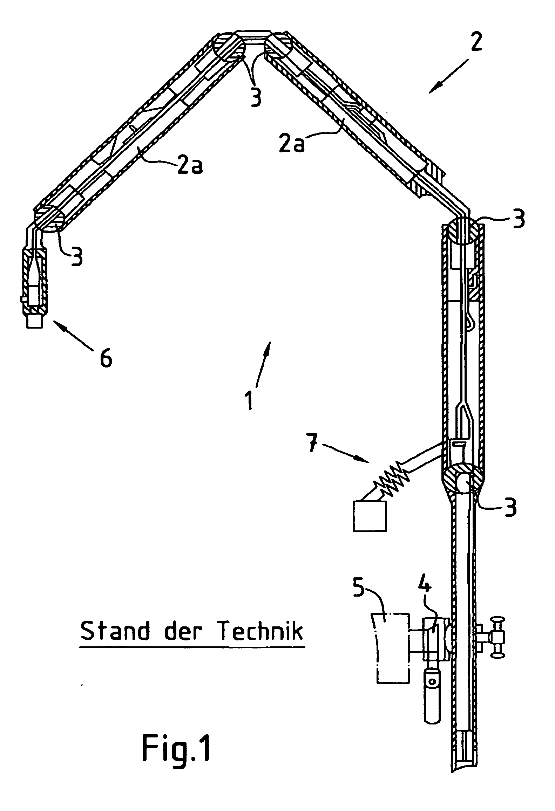

Die Abbildung Fig. 1 zeigt eine Haltevorrichtung 1 für medizinische Instrumente gemäß dem Stand der Technik.

Diese Haltevorrichtung 1 besteht im Wesentlichen aus einem aus mehreren Tragarmteilen 2a bestehenden Tragarm 2, wobei die einzelnen Tragarmteile 2a des Tragarms über als Kugelgelenke 3 ausgebildete Gelenke 3 relativ zueinander verschwenkbar miteinander verbunden sind.

Derartige Haltevorrichtungen 1 sind bei der Durchführung chirurgischer Eingriffe häufig erforderlich, um medizinische Instrumente verschiedenster Art, wie beispielsweise Retraktoren, Mikroskope, Kameras oder Endoskope, über einen längeren Zeitraum in einer bestimmten Position zu halten. Durch die gelenkige Ausgestaltung der Haltevorrichtung 1 ist es für den Chirurgen möglich, das medizinische Instrument exakt zu positionieren und die eingestellte Position der Haltevorrichtung 1 durch Arretieren des Gelenks 3 bzw. der Gelenke 3 zu fixieren. Neben der endoskopischen Chirurgie finden derartige Haltevorrichtungen 1 auch in der offenen Chirurgie Anwendung.

Im Bereich seines proximalen Endes ist der Tragarm 2 über eine Einspannvorrichtung 4, beispielsweise am Operationstisch 5, festlegbar. Am distalen Ende weist der Tragarm 2 eine Instrumentenaufnahme 6 zur Aufnahme des über die Haltevorrichtung 1 zu positionierenden medizinischen Instruments auf.

Alternativ zu dem in Fig. 1 dargestellten Aufbau des Tragarms 2 aus mehreren hintereinander gekoppelten Tragarmteilen 2a, die über Gelenke 3 miteinander verbunden sind, ist es selbstverständlich auch möglich den Tragarm 2 mehrarmig so auszugestalten, dass ausgehend von einem Gelenk 3 mehrere Tragarmteile 2a in verschiedene Richtungen zeigen. Diese Tragarmteile 2a ihrerseits können wieder über Gelenke 3 mit weiteren Tragarmteilen 2a gekoppelt sein und an ihren distalen Enden jeweils mit Instrumentenaufnahmen 6 zur Aufnahme der über die Haltevorrichtung 1 zu positionierenden medizinischen Instrumente ausgestattet sein.

Weiterhin ist es alternativ zu der in Fig. 1 dargestellten Lagerung des Tragarms 2 an einem Operationstisch 5 auch möglich, den Tragarm 2 verschwenkbar an der Decke, an einer Wand oder am Boden zu lagern.

Um dem Operateur die Bedienung einer solchen mit einem medizinischen Instrument bestückten Haltevorrichtung 1 zu erleichtern, ist die Haltevorrichtungen 1 mit einem Gewichtsentlastungssystem 7 zur Kompensierung der Tragarmlast der Haltevorrichtung 1 versehen. Das dargestellte Gewichtsentlastungssystem 7 in der Form von Federpaketen dient dazu, das Eigengewicht des Tragarms 2 nebst den daran festgelegten medizinischen Instrumenten auszubalancieren, so dass der Operateur die Haltevorrichtung 1 nahezu kraftfrei in jede gewünschte Position verschwenken kann.

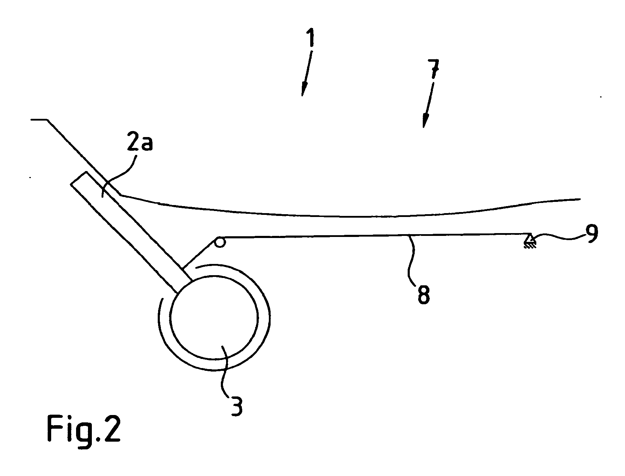

Der Aufbau des erfindungsgemäßen Gewichtsentlastungssystems 7 ist den Abbildungen Fig. 2 bis 4 zu entnehmen.

Fig. 2 zeigt anhand einer Prinzipskizze schematisch den Aufbau und die Wirkungsweise des nachfolgend näher beschriebenen Gewichtsentlastungssystems 7. Die Skizze zeigt ein Gelenk 3 und einen mit dem Gelenk 3 verschwenkbar verbundenen Tragarmteil 2a. Bei dem Gelenk 3 kann es sich beispielsweise um das Gelenk 3 handeln, über das die Haltevorrichtung 1 verschwenkbar an einem Operationstisch 5, an der Decke, an einer Wand oder am Boden gelagert ist.

Das dargestellte Gewichtsentlastungssystem 7 besteht aus einem als Seilzug 8 ausgebildeten Rückstellelement 8, das mit einem Ende an dem über das Gelenk 3 verschwenkbaren Tragarmteil 2a gelagert ist und mit dem anderen Ende an einem ortsfesten Widerlager 9 gelagert ist.

Der Seilzug 8 selber besteht aus einem Draht aus einer eine Plateau-Spannung aufweisenden Formgedächtnis-Legierung, wie beispielsweise NiTiNol. Eine solche Legierung zeichnet sich dadurch aus, dass sie einerseits innerhalb eines dem jeweiligen Werkstoff eigenen Längenänderungsbereichs ohne zusätzlichen oder zumindest ohne relevanten zusätzlichen Kraftaufwand (Zugspannung) dehnbar sind und andererseits nach dem Nachlassen der Zugspannung selbsttätig wieder ihre ursprüngliche Form und Länge einnehmen.

Bei der intermetallischen Legierung NiTiNol liegt das Spannungsplateau im Bereich einer Dehnung von 1 % bis 8 %.

Die Dimensionierung des das Gewichtsentlastungssystem 7 bildenden Seilzuges 8 sowie der Lagerungspunkt des Seilzuges 8 am verschwenkbaren Tragarmteil 2a sind so bemessen, dass der Seilzug 8 in der nicht ausgelenkten Ruhestellung des Tragarms 2 durch das Eigengewicht des Tragarms 2 sowie der daran angeordneten medizinischen Instrumente bis zum Erreichen des Spannungsplateaus vorgespannt ist, so dass die weitere Dehnung des Seilzuges 8 bis zum Ende des Spannungsplateaus ohne zusätzlichen Kraftaufwand erfolgen kann.

Für die Darstellung gemäß Fig. 2 bedeutet dies, dass beim Auslenken des Tragarmteils 2a weiter nach links eine zusätzliche Zugbelastung auf den Seilzug 8 ausgeübt wird, diese Zugbelastung aber in die kraftfreie Längenänderung innerhalb des Spannungsplateaus umgesetzt wird, so dass der die Haltevorrichtung 1 verstellende Operateur das verschwenken des Tragarms 2 nahezu kraftfrei vornehmen kann. Sobald der Tragarm 2 wieder in die Ausgangsstellung zurück geschwenkt wird, zieht sich der Draht des Seilzuges 8 aufgrund der Formgedächtnis-Eigenschaft der verwendeten Legierung wieder auf seine ursprüngliche Länge zusammen.

Dieser reversibel wiederholbare Vorgang ermöglicht bei kompakter Bauweise und geringem Eigengewicht die Herstellung eines Gewichtsentlastungssystems 7, das, beispielsweise zu Reinigungszwecken, leicht demontierbar und wieder montierbar ist.

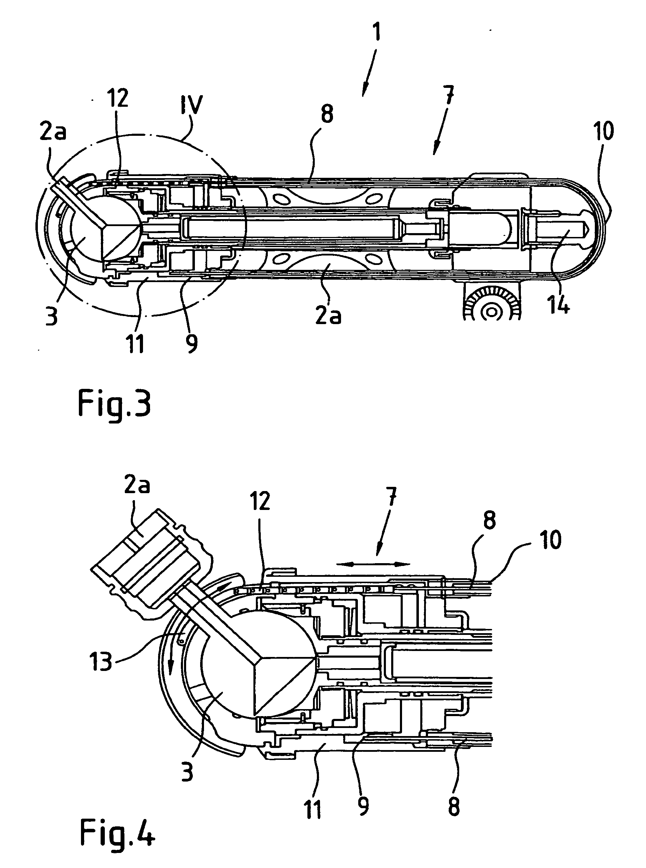

Die Abbildungen Fig. 3 und 4 zeigen eine konkrete technische Ausgestaltung des zuvor anhand der Prinzipskizze gemäß Fig. 2 beschriebenen Gewichtsentlastungssystems 7.

Fig. 3 zeigt einen Schnitt durch ein Tragarmteil 2a einer Haltevorrichtung 1 für medizinische Instrumente. Am auf der Zeichnung linken Ende des Tragarmteils 2a ist ein als Kugelgelenk ausgebildetes Gelenk 3 angeordnet, über das ein angrenzender Tragarmteil 2a gegenüber dem dargestellten Tragarmteil 2a verschwenkbar ist.

Der das Gewichtsentlastungssystem 7 bildende Seilzug 8 ist bei der dargestellten Ausführungsform in einem Führungsrohr 10 angeordnet, das seinerseits axialverschiebbar im Gehäuse 11 des Tragarmteils 2a gelagert ist.

Da das Spannungsplateau, in dem der Seilzug 8 kraftfrei dehnbar ist, eine prozentuale Längenänderung der Gesamtlänge des Seilzuges 8 darstellt, fällt die absolute Längenänderung um so größer aus, je länger der den Seilzug 8 bildende Draht ist. Aus diesem Grunde ist der Seilzug 8 so im Gehäuse 11 des Tragarmteils 2a angeordnet, dass er fast die gesamte Außenkontur des Tragarmteils 2a in Längsrichtung des Tragarmteils 2a umschlingt.

Das Widerlager 9 bildend ist der Seilzug 8 auf der Zeichnung unten mit einem Ende ortsfest am Gehäuse 10 des Tragarmteils 2a gelagert. Mit dem anderen Ende ist der Seilzug 8 das Gelenk 3 überbrückend mit dem über das Gelenk 3 verschwenkbaren Tragarmteil 2a gekoppelt, so dass beide Enden des Seilzuges 8 an zwei verschiedenen Tragarmteilen 2a gelagert sind.

Alternativ zu der dargestellten Ausführungsform ist es selbstverständlich auch möglich, das Widerlager 9 nicht an einem Tragarmteil 2a, sondern beispielsweise an einer Einspannvorrichtung 4 ausgebildet ist, über die die Haltevorrichtung 1 verschwenkbar am Operationstisch 5, der Decke, einer Wand oder am Boden gelagert ist.

Bei der dargestellten Ausführungsform ist der Seilzug 8 auf Seiten des verschwenkbaren Tragarmteils 2a nicht direkt, sondern über ein biegsames Element 12 in Form einer Gliederkette 12 am verschwenkbaren Tragarmteil 2a gelagert, wodurch eine gleichmäßige und ruckfreie Übertragung der von dem verschwenkbaren Tragarmteil 2a ausgeübten Kräfte auf den das Gewichtsentlastungssystem7 bildenden Seilzug 8 gewährleistet wird.

Die Kopplung der Gliederkette 12 mit dem verschwenkbaren Tragarmteil 2a erfolgt gemäß dieser, insbesondere in Fig. 4, dargestellten Ausgestaltungsform über einen Ring 13, der einerseits beweglich mit der Gliederkette 12 verbunden ist und andererseits auf den verschwenkbaren Tragarmteil 2a aufschiebbar ist.

Damit der Seilzug 8 als Gewichtsentlastungssystem 7 das Eigengewicht der Haltevorrichtung 1 mitsamt der daran angeordneten medizinischen Instrumente ausbalancieren kann, wird nach der Montage des Tragarms 2 der den Seilzug 8 bildende Draht über eine Einstellmechanik 14 bis zum Erreichen des Spannungsplateaus vorgespannt.

Die Einstellmechanik 14 ist so in dem mit dem Führungsrohr 10 versehenen Tragarmteil 2a angeordnet, dass über die Einstellmechanik 14 das axialverschiebbar im Gehäuse 11 gelagerte Führungsrohr 10 ―auf der Zeichnung Fig. 3 nach rechts- fort vom Widerlager 9 gedrückt wird. Da der Seilzug 8 mit einem Ende ortsfest am Widerlager 9 gelagert ist und auf das andere Ende das Eigengewicht des Tragarms 2 wirkt, bewirkt das Verschieben des Führungsrohres 10 mit dem darin angeordneten Seilzug 8 eine Dehnung des Seilzuges 8.

Die Dimensionierung des das Gewichtsentlastungssystem 7 bildenden Seilzuges 8 sowie Lagerungspunkt des Seilzuges 8 am verschwenkbaren Tragarmteil 2a sind so bemessen, dass der Seilzug 8 in der nicht ausgelenkten Ruhestellung des Tragarms 2 durch das Eigengewicht des Tragarms 2 sowie der daran angeordneten medizinischen Instrumente bis zum Erreichen des Spannungsplateaus vorgespannt ist, so dass die weitere Dehnung des Seilzuges 8 bis zum Ende des Spannungsplateaus ohne zusätzlichen Kraftaufwand erfolgen kann. Die Feineinstellung bis zum Erreichen des Spannungsplateaus erfolgt über die Einstellmechanik 14.

Für die Darstellung gemäß Fig. 3 und 4 bedeutet dieses Vorspannen des Seilzuges 8 bis zum Erreichen des Spannungsplateaus des verwendeten NiTiNol-Drahtes, dass beim Auslenken des verschwenkbaren Tragarmteils 2a weiter nach links zwar eine zusätzliche Zugbelastung auf den Seilzug 8 ausgeübt wird, diese Zugbelastung aber in eine kraftfreie Längenänderung des Seilzuges 8 innerhalb des Spannungsplateaus umgesetzt wird, so dass der die Haltevorrichtung 1 verstellende Operateur das verschwenken des Tragarms 2 nahezu kraftfrei vornehmen kann. Sobald der Tragarm 2 wieder in die Ausgangsstellung zurück geschwenkt wird, zieht sich der Draht des Seilzuges 8 aufgrund der Formgedächtnis-Eigenschaft der verwendeten NiTiNol-Legierung wieder auf seine ursprüngliche Länge zusammen.

Eine solchermaßen mit einem Gewichtsentlastungssystem 7 versehene Haltevorrichtung 1 für medizinische Instrumente zeichnet sich dadurch aus, dass der reversibel wiederholbare Vorgang der kraftfreien Längenänderung die Herstellung eines Gewichtsentlastungssystems 7 mit einer kompakten Bauweise und einem geringen Eigengewicht ermöglicht, so dass die Haltevorrichtung 1, beispielsweise zu Reinigungszwecken, leicht demontierbar und wieder montierbar ist und auch vollständig autoklavierbar ist.

### Bezugszeichenliste

- 1: Haltevorrichtung
- 2: Tragarm
- 2a: Tragarmteil
- 3: Gelenk / Kugelgelenk
- 4: Einspannvorrichtung
- 5: Operationstisch
- 6: Instrumentenaufnahme
- 7: Gewichtsentlastungssystem
- 8: Rückstellelement / Seilzug
- 9: Widerlager
- 10: Führungsrohr
- 11: Gehäuse
- 12: biegsames Element / Gliederkette
- 13: Ring
- 14: Einstellmechanik

## Patentansprüche

1. Haltevorrichtung für medizinische Instrumente, mit einem Tragarm (2), an dem mindestens ein medizinisches Instrument festlegbar ist, mit mindestens einem Gelenk (3) zur Positionierung des Tragarms (2) sowie mit einem Gewichtsentlastungssystem (7) zur Kompensierung der Tragarmlast,
**dadurch gekennzeichnet,**
**dass** das Gewichtsentlastungssystem (7) als mit dem Tragarm (2) gekoppeltes Rückstellelement (8) aus einem im Wesentlichen eine Plateau-Spannung aufweisenden Material ausgebildet ist.

2. Haltevorrichtung nach Anspruch 1, **dadurch gekennzeichnet, dass** das eine Plateau-Spannung aufweisende Material des Rückstellelements (8) eine CuZnAl-, FeNiCoTi- oder NiTi-Legierung ist.

3. Haltevorrichtung nach Anspruch 2, **dadurch gekennzeichnet, dass** das eine Plateau-Spannung aufweisende Material eine Formgedächtnis-Legierung ist.

4. Haltevorrichtung nach Anspruch 3, **dadurch gekennzeichnet, dass** die das Rückstellelement (8) bildende Formgedächtnis-Legierung eine NiTi-Legierung, insbesondere NiTiNol, ist.

5. Haltevorrichtung nach einem der Ansprüche 1 bis 4, **dadurch gekennzeichnet, dass** das Rückstellelement (8) durch die Tragarmlast bis auf das Erreichen der Plateau-Spannung vorgespannt ist.

6. Haltevorrichtung nach einem der Ansprüche 1 bis 5, **dadurch gekennzeichnet, dass** das Rückstellelement (8) das mindestens eine Gelenk (3) überspannend mit dem Tragarm (2) gekoppelt ist.

7. Haltevorrichtung nach einem der Ansprüche 1 bis 6, **dadurch gekennzeichnet, dass** das Rückstellelement (8) als Seilzug (8) ausgebildet ist.

8. Haltevorrichtung nach einem der Ansprüche 1 bis 6, **dadurch gekennzeichnet, dass** das Rückstellelement (8) als Federelement ausgebildet ist.

9. Haltevorrichtung nach Anspruch 7, **dadurch gekennzeichnet, dass** der Seilzug (8) mit einem Ende an einem ersten Tragarmteil (2a) gelagert ist und mit dem anderen Ende an einem über das mindestens eine Gelenk (3) gegenüber dem ersten Tragarmteil (2a) verstellbaren zweiten Tragarmteil (2a) gelagert ist.

10. Haltevorrichtung nach Anspruch 9 **dadurch gekennzeichnet, dass** der Seilzug (8) über ein biegsames Element (12) an dem verstellbaren Tragarmteil (2a) gelagert ist.

11. Haltevorrichtung nach Anspruch 10, **dadurch gekennzeichnet, dass** das biegsame Element (12) als Gliederkette ausgebildet ist.

12. Haltevorrichtung nach einem der Ansprüche 9 bis 1, **dadurch gekennzeichnet, dass** der Seilzug (8) in einem Führungsrohr (10) gelagert ist.

13. Haltevorrichtung nach Anspruch 12, **dadurch gekennzeichnet, dass** das Führungsrohr (10) axialverschiebbar am Tragarm (2) gelagert ist.

14. Haltevorrichtung nach Anspruch 5 und 13, **dadurch gekennzeichnet, dass** die Vorspannung des Seilzugs (8) über das axialverschiebbare Führungsrohr (10) einstellbar ist.

## Claims

1. Holding device for medical instruments, with a bracket (2) on which at least one medical instrument can be secured, with at least one hinge (3) for positioning the bracket (2), and with a weight-relieving system (7) to compensate the bracket load, **characterized in that** the weight-relieving system (7) is designed as a resetting element (8) which is coupled to the bracket (2) and is made of a material having substantially a plateau stress.

2. Holding device according to Claim 1, **characterized in that** the material of the resetting element (8) having a plateau stress is a CuZnAl, FeNiCoTi or NiTi alloy.

3. Holding device according to Claim 2, **characterized in that** the material having a plateau stress is a shape-memory alloy.

4. Holding device according to Claim 3, **characterized in that** the shape-memory alloy forming the resetting element (8) is an NiTi alloy, in particular NiTiNol.

5. Holding device according to one of Claims 1 to 4, **characterized in that** the resetting element (8) is pre-tensioned by the bracket load until the plateau stress is reached.

6. Holding device according to one of Claims 1 to 5, **characterized in that** the resetting element (8), bridging the at least one hinge (3), is coupled to the bracket (2).

7. Holding device according to one of Claims 1 to 6, **characterized in that** the resetting element (8) is designed as a cable pull (8).

8. Holding device according to one of Claims 1 to 6, **characterized in that** the resetting element (8) is designed as a spring element.

9. Holding device according to Claim 7, **characterized in that** the cable pull (8) is mounted with one end on a first bracket part (2a) and with the other end on a second bracket part (2a) that is adjustable with respect to the first bracket part (2a) via the at least one hinge (3).

10. Holding device according to Claim 9, **characterized in that** the cable pull (8) is mounted on the adjustable bracket part (2a) via a flexible element (12).

11. Holding device according to Claim 10, **characterized in that** the flexible element (12) is designed as a link chain.

12. Holding device according to one of Claims 9 to 11, **characterized in that** the cable pull (8) is mounted in a guide tube (10).

13. Holding device according to Claim 12, **characterized in that** the guide tube (10) is mounted axially displaceably on the bracket (2).

14. Holding device according to Claims 5 and 13, **characterized in that** the pre-tensioning of the cable pull (8) is adjustable via the axially displaceable guide tube (10).

## Revendications

1. Dispositif de retenue pour instruments médicaux, comprenant un bras de support (2), sur lequel peut être fixé au moins un instrument médical, comprenant au moins une articulation (3) pour le positionnement du bras de support (2) et comprenant un système de décharge de poids (7) pour compenser la charge du bras de support,
**caractérisé en ce que**
le système de décharge de poids (7) est réalisé en tant qu'élément de rappel (8) accouplé au bras de support (2) à partir d'un matériau présentant essentiellement une contrainte de plateau.

2. Dispositif de retenue selon la revendication 1, **caractérisé en ce que** le matériau de l'élément de rappel (8) présentant une contrainte de plateau est un alliage de CuZnAl, de FeNiCoTi ou de NiTi.

3. Dispositif de retenue selon la revendication 2, **caractérisé en ce que** le matériau présentant une contrainte de plateau est un alliage à mémoire de forme.

4. Dispositif de retenue selon la revendication 3, **caractérisé en ce que** l'alliage à mémoire de forme formant l'élément de rappel (8) est un alliage de NiTi, en particulier du NiTiNol.

5. Dispositif de retenue selon l'une quelconque des revendications 1 à 4, **caractérisé en ce que** l'élément de rappel (8) est précontraint par la charge du bras de support jusqu'à l'obtention de la contrainte de plateau.

6. Dispositif de retenue selon l'une quelconque des revendications 1 à 5, **caractérisé en ce que** l'élément de rappel (8) est accouplé au bras de support (2) en surmontant l'au moins une articulation (3).

7. Dispositif de retenue selon l'une quelconque des revendications 1 à 6, **caractérisé en ce que** l'élément de rappel (8) est réalisé sous forme de câble Bowden (8).

8. Dispositif de retenue selon l'une quelconque des revendications 1 à 6, **caractérisé en ce que** l'élément de rappel (8) est réalisé sous forme d'élément de ressort.

9. Dispositif de retenue selon la revendication 7, **caractérisé en ce que** le câble Bowden (8) est supporté par une extrémité au niveau d'une première partie de bras de support (2a) et par l'autre extrémité au niveau d'une deuxième partie de bras de support (2a) réglable par le biais de l'au moins une articulation (3) par rapport à la première partie de bras de support (2a).

10. Dispositif de retenue selon la revendication 9, **caractérisé en ce que** le câble Bowden (8) est supporté par le biais d'un élément flexible (12) au niveau de la partie de bras de support réglable (2a).

11. Dispositif de retenue selon la revendication 10, **caractérisé en ce que** l'élément flexible (12) est réalisé sous forme de chaîne à maillons.

12. Dispositif de retenue selon l'une quelconque des revendications 9 à 11, **caractérisé en ce que** le câble Bowden (8) est supporté dans un tube de guidage (10).

13. Dispositif de retenue selon la revendication 12, **caractérisé en ce que** le tube de guidage (10) est supporté de manière déplaçable axialement sur le bras de support (2).

14. Dispositif de retenue selon la revendication 5 et 13, **caractérisé en ce que** la précontrainte du câble Bowden (8) peut être ajustée par le biais du tube de guidage déplaçable axialement (10).
